# FASCICULE DE BREVET EUROPEEN

(11) **EP 1 348 684 B1**
(45) Date de publication et mention de la délivrance du brevet: **08.03.2006**
(21) Numéro de dépôt: 03290879.0
(22) Date de dépôt: 09.04.2003
(51) Int. Cl.: C07B 57/00, C07D 209/42

(54) **Nouveau procédé de synthèse de l'acide (2S)-indoline-2-carboxilique, et application à la synthèse du perindopril**
Verfahren zur Synthese von (2S)-Indolin-2-carbonsäure, und Verwendung in der Synthese von Perindopril
Method for synthesis of (2S)-indoline-2-carboxylic acid; and use in the synthesis of perindopril

(43) Date de publication de la demande: 01.10.2003
(73) Titulaire: Les Laboratoires Servier, 92415 Courbevoie Cedex (FR)
(72) Inventeur: Souvie, Jean-Claude, 76600 Le Havre (FR); Lecouve, Jean-Pierre, 76600 Le Havre (FR)

(56) Documents cités:
- EP-A- 0 308 341
- EP-A- 0 468 592
- EP-A- 0 703 212
- DE-A- 3 727 411
- US-A- 5 220 053
- US-A- 5 367 091

## Description

La présente invention concerne un procédé de synthèse industrielle de l'acide (2S)-indoline-2-carboxylique, et son application à la synthèse industrielle du perindopril et de ses sels pharmaceutiquement acceptables.

Plus spécifiquement, la présente invention concerne un nouveau procédé de synthèse industrielle de l'acide (2S)-indoline-2-carboxylique de formule (1) :

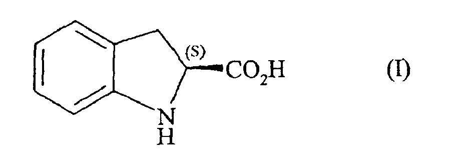

Le composé de formule (1) obtenu selon le procédé de l'invention est utile dans la synthèse du perindopril de formule (II) : 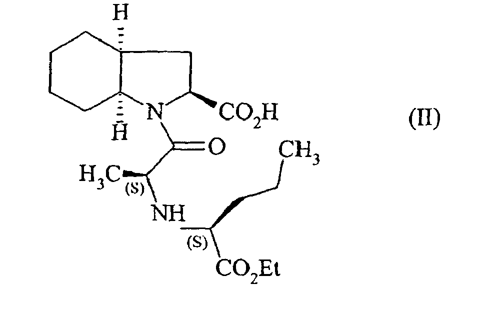 ainsi que dans celle de ses sels pharmaceutiquement acceptables.

Le perindopril, ainsi que ses sels, possèdent des propriétés pharmacologiques intéressantes.

Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir accéder à l'intermédiaire de formule (I) avec un procédé de synthèse industrielle performant, permettant l'obtention sélective de l'énantiomère (S) avec une excellente pureté et un très bon rendement.

Quelques méthodes de préparation du composé de formule (I) sont déjà connues.

Ainsi, les brevets EP 0 308 339 et EP 0 308 341 décrivent l'obtention de l'acide (2S)-indoline-2-carboxylique par dédoublement de l'acide indoline 2-carboxylique racémique à l'aide de (R)-α-méthylbenzylamine. Le sel de (R)-α-méthylbenzylamine de l'acide (2S)-indoline-2-carboxylique est isolé par cristallisation fractionnée, puis acidifié pour conduire au composé de formule (I).
Cette méthode présente l'avantage d'utiliser une matière première et des réactifs peu coûteux et très aisément accessibles.
Par contre, elle ne conduit au composé de formule (I) qu'avec un rendement de 35 %.

La Demanderesse a présentement mis au point un procédé de synthèse du composé de formule (I), dans lequel l'isomère (2R) qui est formé au cours de la réaction de dédoublement est recyclé. Le procédé ainsi mis au point permet d'obtenir le composé de formule (I) avec un rendement à partir de l'acide indoline 2-carboxylique racémique allant de 50 % à 70 % selon le nombre de recyclages effectué.

Plus spécifiquement, la présente invention concerne un procédé de synthèse industrielle du composé de formule (I), caractérisé en ce que l'on fait réagir l'acide indoline 2-carboxylique racémique de formule (III) : 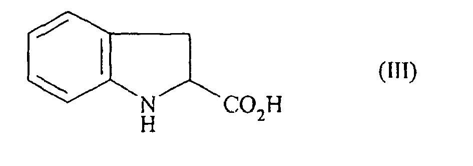 avec une amine chirale,
pour conduire au sel de formule (IV) : 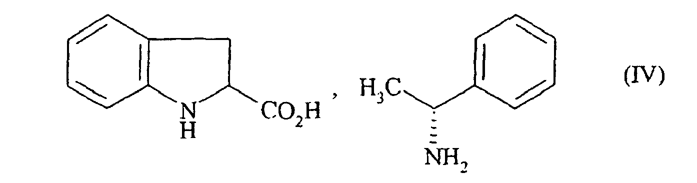
que l'on filtre, et isole ainsi :
- d'une part l'isomère (2S) de formule (IV a) : 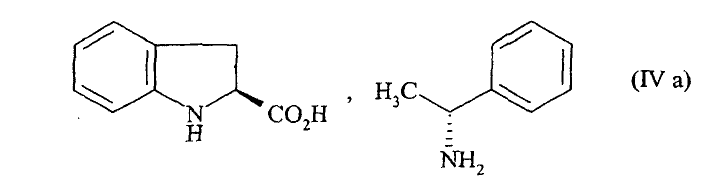 sous forme de cristaux,
   composé de formule (IV a) qui est ensuite traité par l'acide chlorhydrique pour conduire au composé de formule (I),
- et d'autre part un mélange majoritairement (2R) de l'isomère (2S) de formule (IV a) et de l'isomère (2R) de formule (IV b) : 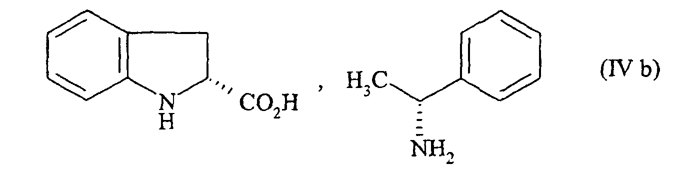 par évaporation du filtrat,
   mélange qui est ensuite traité par l'acide chlorhydrique, pour conduire à un mélange majoritairement (2R) de l'acide (2R)-indoline 2-carboxylique et de l'acide (2S) indoline 2-carboxylique,
   que l'on racémise par réaction avec de la soude,
   à une température comprise entre 140 et 200°C,
   sous une pression comprise entre 5 et 15 bars,
   pour conduire après isolement au composé de formule (III), sur lequel on réitère la
   série d'opérations précédemment décrites,
puis, après avoir effectué entre 2 et 6 cycles, on rassemble toutes les fractions constituées du composé de formule (I).

Le composé de formule (I) est ainsi obtenu avec un rendement allant de 50 % à 70 % suivant le nombre de cycles effectué.

Sa pureté chimique et énantiomérique est très bonne, ce qui rend son emploi particulièrement avantageux dans la synthèse du perindopril de formule (II).

A titre d'illustration, l'hydrogénation catalytique du composé de formule (I) obtenu selon le procédé de l'invention, suivie du couplage de l'acide (2S, 3aS, 7aS)-perhydroindole 2-carboxylique ainsi obtenu avec le composé de formule (VI) : 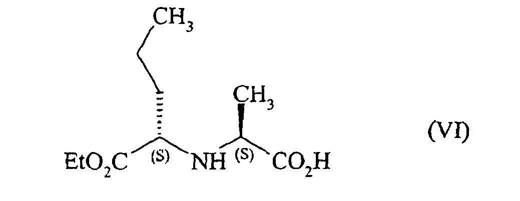 permet d'obtenir le perindopril de formule (II) avec une pureté et un rendement très satisfaisants.

La présente invention concerne également une variante du procédé précédent, dans laquelle on met en réaction l'acide (2R)-indoline 2-carboxylique de formule (V) : 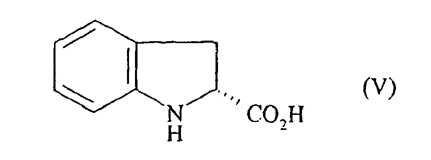 que l'on racémise par réaction avec de la soude,
à une température comprise entre 140 et 200°C,
sous une pression comprise entre 5 et 15 bars,
pour conduire après isolement au composé de formule (III) : 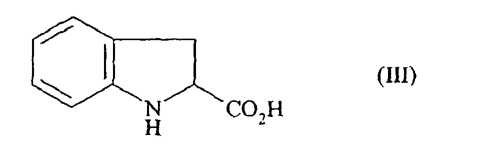 que l'on met en réaction avec une amine chirale,
pour conduire au sel de formule (IV) : 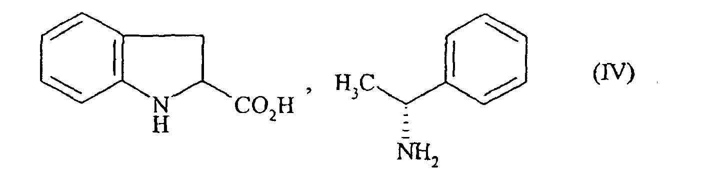
que l'on filtre, et isole ainsi :
- d'une part l'isomère (2S) de formule (IV a) : 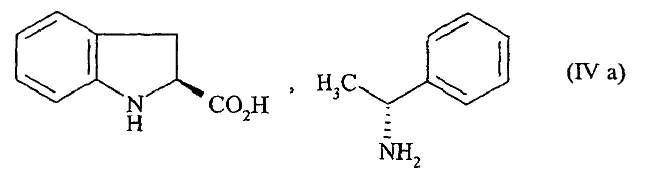 sous forme de cristaux,
   composé de formule (IV a) qui est ensuite traité par l'acide chlorhydrique pour conduire au composé de formule (I),
- et d'autre part un mélange majoritairement (2R) de l'isomère (2S) de formule (IV a) et de l'isomère (2R) de formule (IV b) : 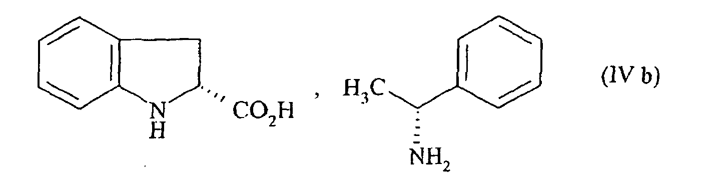 par évaporation du filtrat,
   mélange qui est ensuite traité par l'acide chlorhydrique, pour conduire à un mélange majoritairement (2R) de l'acide (2R)-indoline 2-carboxylique et de l'acide (2S) indoline 2-carboxylique,
   sur lequel on réitère, lorsqu'on le souhaite, la série d'opérations précédemment décrites,
puis, après avoir effectué entre 1 et 6 cycles, on rassemble toutes les fractions constituées du composé de formule (1).

Parmi les amines chirales utilisables dans le procédé selon l'invention ou sa variante, on peut citer à titre non limitatif la (R)-α-méthylbenzylamine, la 1-(1-naphtyl)-éthylamine, l'éphédrine, la α-chymotrypsine, la sec-butylamine, le 1-amino-2-méthylbutane, la N,N-diméthyl-1-phényléthylamine, la 1-cyclohexyléthylamine, la cyclosérine, la 2-(méthoxyméthyl)-pyrrolidine, le α-diméthylamino-ε-caprolactame, l'isobornylamine, la 1-(4-nitrophényl)-éthylamine, le α-amino-ε-caprolactame, le 2-amino-1-butanol, le 1-amino-2-propanol, la cinchonidine, la cinchonine, la N-méthyl-éphédrine, le phénylalaninol, la quinidine, le valinol, le α-phényl-glycinol, le leucinol.

L'amine chirale préférée est la (R)-α-méthylbenzylamine.

Par mélange majoritairement (2R), on entend un mélange d'isomères (2R) et (2S) dans lequel l'isomère (2R) est majoritaire.

L'exemple ci-dessous illustre l'invention mais ne la limite en aucune façon.

### EXEMPLE : Acide (2S) indoline 2-carboxylique

### Stade A : Dédoublement de l'acide indoline 2-carboxylique racémique

3,7 kg de (R)-α-méthylbenzylamine sont ajoutés à une solution de 5 kg d'acide indoline 2-carboxylique dans l'éthanol, puis le mélange est agité pendant 2h et filtré.

### Stade A₁ : Acide (2S) indoline 2-carboxylique

Le précipité blanc recueilli dans le stade A est recristallisé dans l'isopropanol, puis dissout dans 13 l d'eau, et 12 1 d'une solution d'acide chlorhydrique 1 N sont ajoutés.
Après 2h d'agitation, le précipité est filtré, puis lavé et séché, pour conduire à l'acide (2S) indoline 2-carboxylique (« 1^{ère} fraction ») sous forme de cristaux (1,80 kg) avec une pureté chimique de 98 % et une pureté énantiomérique supérieure à 99,5 %.

### Stade A₂ : Acide indoline 2-carboxylique (mélange majoritairement (2R))

Le filtrat recueilli dans le stade A est évaporé, et le résidu obtenu est dissout dans 13 1 d'eau, puis 12 l d'une solution d'acide chlorhydrique 1 N sont ajoutés.
Après 2h d'agitation, le précipité est filtré, puis lavé et séché, pour conduire à l'acide indoline 2-carboxylique sous forme de mélange majoritairement (2R) des énantiomères (2R) et (2S) (2,6 kg).

### Stade B : Racémisation

Dans un autoclave, charger le précipité obtenu au stade A₂ (2,6 kg), puis 12 l d'eau et 3,1 l d'une solution de soude 8,65 N, puis porter à 170°C pendant 3h sous une pression de 7 bars.
Le mélange réactionnel est ensuite ramené à température ambiante, puis transvasé dans un réacteur, puis de l'acide chlorhydrique concentré est ajouté jusqu'à pH=3,4, en maintenant la température entre 20 et 25°C.

Le mélange est ensuite agité pendant lh, puis le précipité est filtré, lavé et séché, pour conduire à l'acide indoline 2-carboxylique racémique avec un rendement de 90 % (2,34 kg).

### Stade C : Recyclage de l'acide indoline 2-carboxylique

L'acide indoline 2-carboxylique racémique obtenu au stade B (2,34 kg) est dédoublé selon le procédé du stade A.
Le sel de (R)-α-méthylbenzylamine de l'acide (2S) indoline 2-carboxylique ainsi formé est isolé puis traité par l'acide chlorhydrique selon le procédé du stade A₁, pour conduire à l'acide (2S) indoline 2-carboxylique (« 2^{ème} fraction ») sous fonne de cristaux (0,84 kg) avec une pureté chimique de 98 % et une pureté énantiomérique supérieure à 99,5 %.
La 1^{ère} fraction, obtenue au stade A₁, et la 2^{ème} fraction, obtenue au stade C, sont ensuite rassemblées.
L'acide (2S) indoline 2-carboxylique est ainsi obtenu avec un rendement global de 52,8 %, une pureté chimique de 98 % et une pureté énantiomérique supérieure à 99,5 %.

## Revendications

1. Procédé de synthèse industrielle de l'acide (2S) indoline 2-carboxylique de formule (1) : 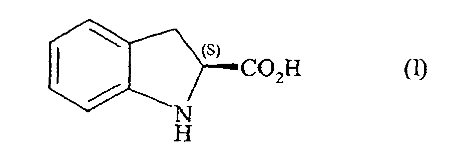
**caractérisé en ce que** l'on fait réagir l'acide indoline 2-carboxylique racémique de formule (III) : 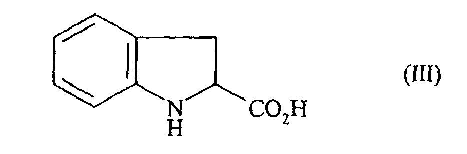 avec une amine chirale,
pour conduire au sel de formule (IV) : 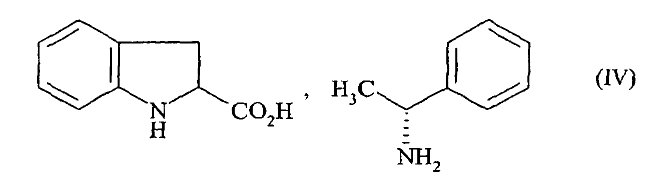 que l'on filtre, et isole ainsi :
• d'une part l'isomère (2S) de formule (IV a) : 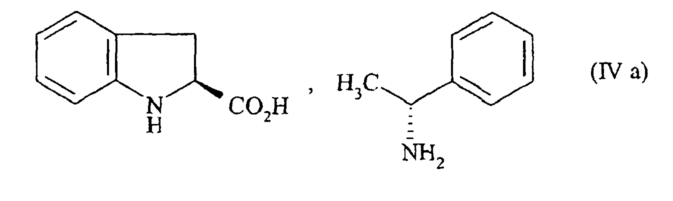 sous forme de cristaux,
composé de formule (IV a) qui est ensuite traité par l'acide chlorhydrique pour conduire au composé de formule (I),
• et d'autre part un mélange majoritairement (2R) de l'isomère (2S) de formule (IV a) et de l'isomère (2R) de formule (IV b) : 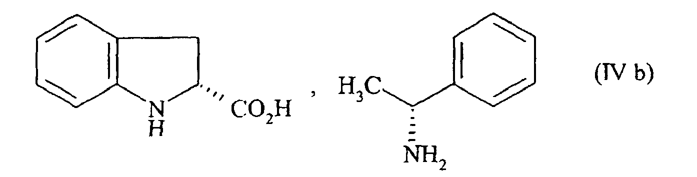 par évaporation du filtrat,
mélange qui est ensuite traité par l'acide chlorhydrique, pour conduire à un mélange majoritairement (2R) de l'acide (2R)-indoline 2-carboxylique et de l'acide (2S) indoline 2-carboxylique,
que l'on racémise par réaction avec de la soude,
à une température comprise entre 140 et 200°C,
sous une pression comprise entre 5 et 15 bars,
pour conduire après isolement au composé de formule (III), sur lequel on réitère la série d'opérations précédemment décrites,
puis, après avoir effectué entre 2 et 6 cycles, on rassemble toutes les fractions constituées du composé de formule (I).

2. Procédé de synthèse industrielle de l'acide (2S) indoline 2-carboxylique de formule (I) : 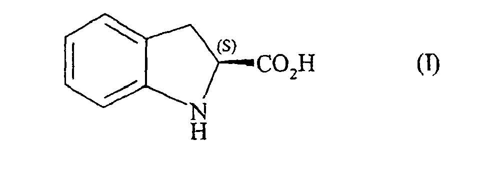
**caractérisé en ce que** l'on met en réaction l'acide (2R)-indoline 2-carboxylique de formule (V) : 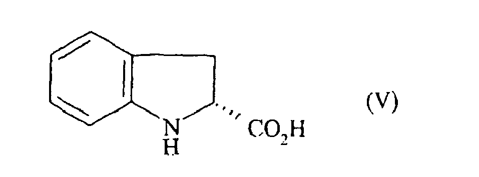 que l'on racémise par réaction avec de la soude,
à une température comprise entre 140 et 200°C,
sous une pression comprise entre 5 et 15 bars,
pour conduire après isolement au composé de formule (III) : 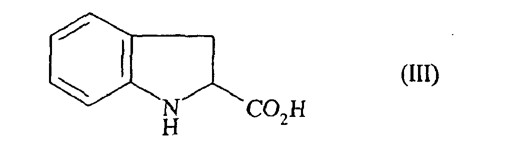 que l'on met en réaction avec une amine chirale,
pour conduire au sel de formule (IV) : 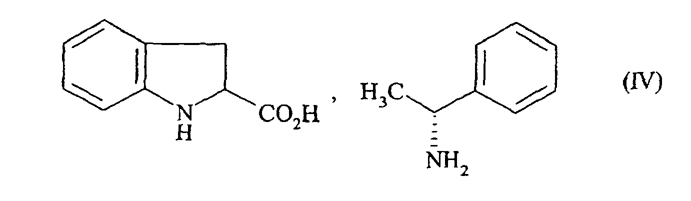
que l'on filtre, et isole ainsi :
• d'une part l'isomère (2S) de formule (N a) : 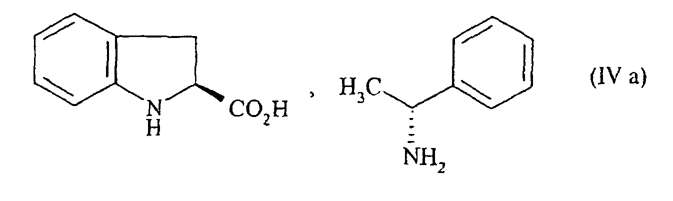 sous forme de cristaux,
composé de formule (IV a) qui est ensuite traité par l'acide chlorhydrique pour conduire au composé de formule (I),
• et d'autre part un mélange majoritairement (2R) de l'isomère (2S) de formule (IV a) et de l'isomère (2R) de formule (IV b) : 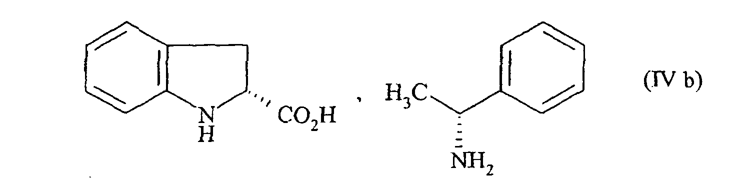 par évaporation du filtrat,
mélange qui est ensuite traité par l'acide chlorhydrique, pour conduire à un mélange majoritairement (2R) de l'acide (2R)-indoline 2-carboxylique et de l'acide (2S) indoline 2-carboxylique,
sur lequel on réitère, lorsqu'on le souhaite, la série d'opérations précédemment décrites,
puis, après avoir effectué entre 1 et 6 cycles, on rassemble toutes les fractions constituées du composé de formule (I).

3. Procédé de synthèse selon l'une quelconque des revendications 1 ou 2, **caractérisé en ce que** l'amine chirale est la (R)-α-méthylbenzylamine.

4. Procédé de synthèse du perindopril ou de ses sels pharmaceutiquemenl acceptables à partir du composé de formule (I), **caractérisé en ce que** ledit composé de formule (I) est obtenu selon le procédé de l'une quelconque des revendications 1 à 3.

## Patentansprüche

1. Verfahren zur technischen Synthese von (2S)-Indolin-2-carbonsäure der Formel (I): 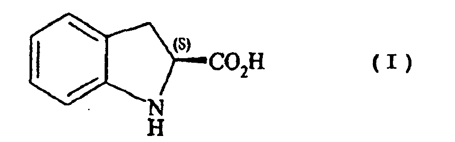 **dadurch gekennzeichnet, daß** man racemische Indolin-2-carbonsäure der Formel (III): 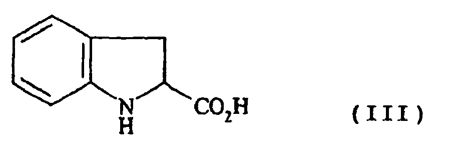 mit einem chiralen Amin umsetzt,
zur Bildung des Salzes der Formel (IV): 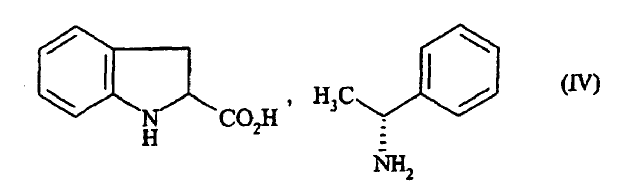
welches man filtriert und in dieser Weise:
• einerseits das (2S)-Isomere der Formel (IV a): 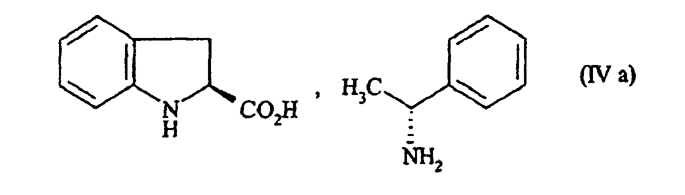 in Form von Kristallen isoliert,
welche Verbindung der Formel (IV a) anschließend mit Chlorwasserstoffsäure behandelt wird zur Bildung der Verbindung der Formel (I),
• und andererseits eine überwiegend (2R)-Mischung des (2S)-Isomeren der Formel (IV a) und des (2R)-Isomeren der Formel (IV b): 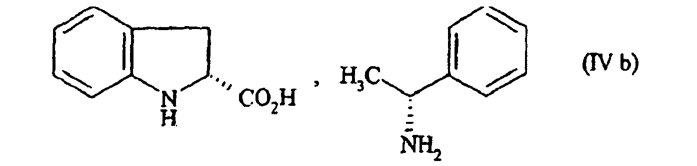 durch Eindampfen des Filtrats isoliert,
welche Mischung man anschließend mit Chlorwasserstoffsäure behandelt zur Bildung einer überwiegend (2R)-Mischung der (2R)-Indolin-2-carbonsäure und der (2S)-Indolin-2-carbonsäure,
welche man durch Reaktion mit Natriumhydroxid
bei einer Temperatur zwischen 140 und 200 °C
bei einem Druck zwischen 5 und 15 bar
recemisiert, so daß man nach der Isolierung die Verbindung der Formel (III) erhält, auf welche man erneut die oben beschriebene Reihe von Verfahrensmaßnahmen anwendet,
und nach der Durchführung von zwischen 2 und 6 Zyklen sämtliche durch die Verbindung der Formel (I) gebildeten Fraktionen vereinigt.

2. Verfahren zur technischen Synthese von (2S)-Indolin-2-carbonsäure der Formel (I): 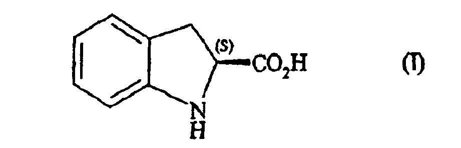 **dadurch gekennzeichnet, daß** man (2R)-Indolin-2-carbonsäure der Formel (V): 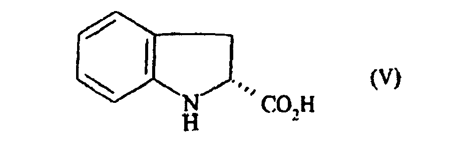 durch Reaktion mit Natriumhydroxid
bei einer Temperatur zwischen 140 und 200 °C,
bei einem Druck zwischen 5 und 15 bar
racemisiert, so daß man nach der Isolierung die Verbindung der Formel (III) erhält: 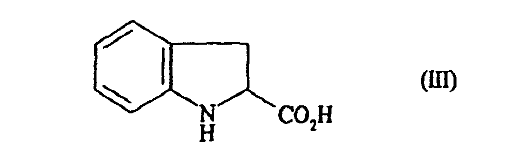 welche man mit einem chiralen Amin umsetzt,
zur Bildung des Salzes der Formel (IV): 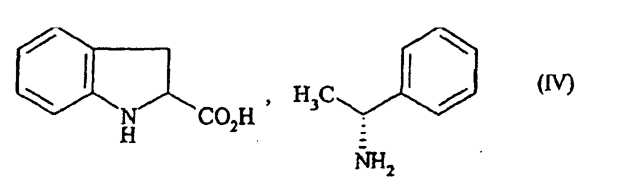
welches man filtriert und in dieser Weise einerseits:
• das (2S)-Isomere der Formel (IV a): 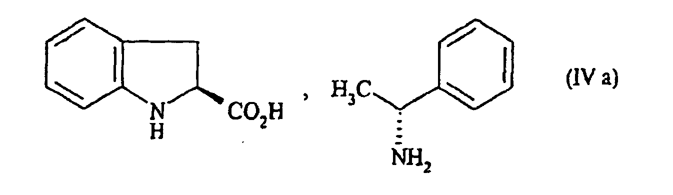 in Form von Kristallen isoliert,
welche Verbindung der Formel (IV a) anschließend mit Chlorwasserstoffsäure behandelt wird zur Bildung der Verbindung der Formel (I),
• und andererseits eine überwiegend (2R)-Mischung des (2S)-Isomeren der Formel (IV a) und des (2R)-Isomeren der Formel (IV b): 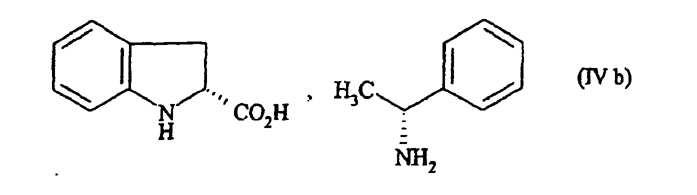 durch Eindampfen des Filtrats isoliert,
welche Mischung anschließend mit Chlorwasserstoffsäure behandelt wird, so daß man eine überwiegend (2R)-Mischung der (2R)-Indolin-2-carbonsäure und der (2S)-Indolin-2-carbonsäure erhält,
auf welche man gewünschtenfalls die oben beschriebenen Verfahrensmaßnahmen wiederholt anwendet,
und man nach der Durchführung von zwischen 1 und 6 Zyklen sämtliche durch die Verbindung der Formel (I) gebildeten Fraktionen vereinigt.

3. Syntheseverfahren nach einem der Ansprüche 1 oder 2, **dadurch gekennzeichnet, daß** das chirale Amin (R)-α-Methylbenzylamin ist.

4. Verfahren zur Synthese von Perindopril oder von seinen pharmazeutisch annehmbaren Salzen ausgehend von der Verbindung der Formel (I), **dadurch gekennzeichnet, daß** man die Verbindung der Formel (I) nach dem Verfahren gemäß einem der Ansprüche 1 bis 3 herstellt.

## Claims

1. Process for the industrial synthesis of (2S)-indoline-2-carboxylic acid of formula (I) : 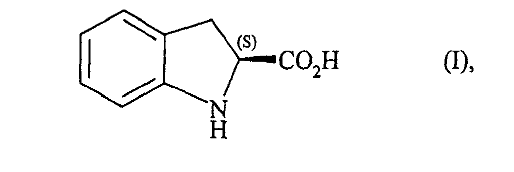 **characterised in that** racemic indoline-2-carboxylic acid of formula (III) : 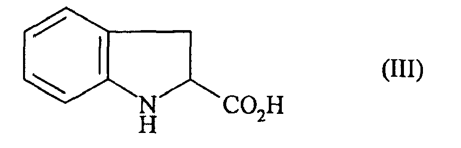 is reacted with a chiral amine
to yield the salt of formula (IV) : 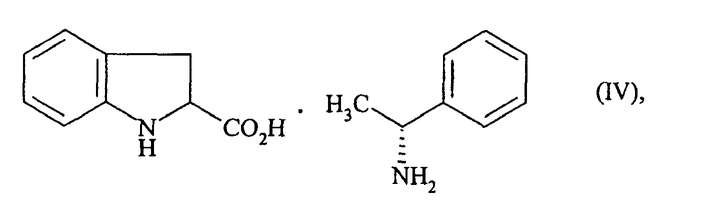
which is filtered off, and there is thus isolated :
• on the one hand the (2S) isomer of formula (IV a) : 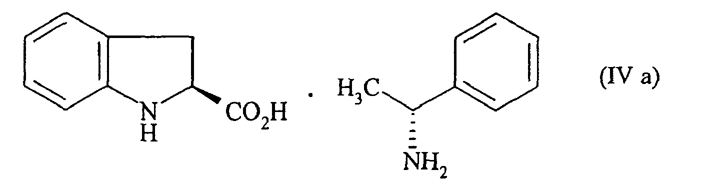 in the form of crystals,
which compound of formula (IV a) is then treated with hydrochloric acid to yield the compound of formula (I),
• and on the other hand, by evaporation of the filtrate, a mixture of the (2S) isomer of formula (IV a) and the (2R) isomer of formula (IV b) in which the (2R) isomer predominates: 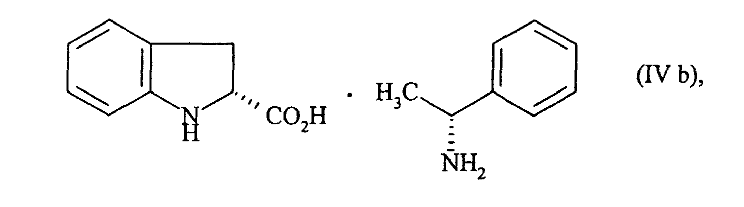 which mixture is then treated with hydrochloric acid to yield a mixture of (2R)-indoline-2-carboxylic acid and (2S)-indoline-2-carboxylic acid in which the (2R) acid predominates,
which is racemised by reaction with sodium hydroxide solution,
at a temperature of from 140 to 200°C,
under a pressure of from 5 to 15 bars,
to yield, after isolation, the compound of formula (III), with which the series of operations described above is repeated,
then, after having carried out from 2 to 6 cycles, all the portions made up of the compound of formula (I) are combined.

2. Process for the industrial synthesis of (2S)-indoline-2-carboxylic acid of formula (I) : 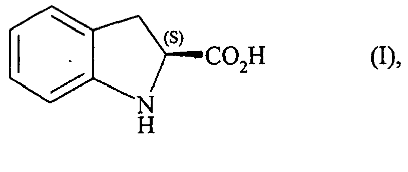 **characterised in that** there is reacted (2R)-indoline-2-carboxylic acid of formula (V) : 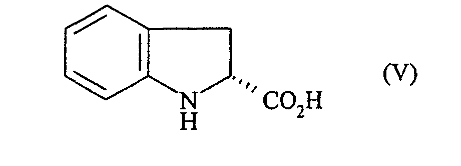 which is racemised by reaction with sodium hydroxide solution,
at a temperature of from 140 to 200°C,
under a pressure of from 5 to 15 bars,
to yield, after isolation, the compound of formula (III) : 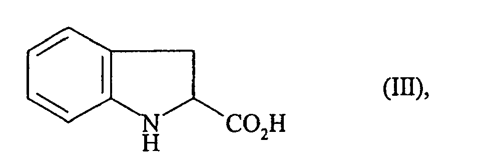
which is reacted with a chiral amine,
to yield the salt of formula (IV) : 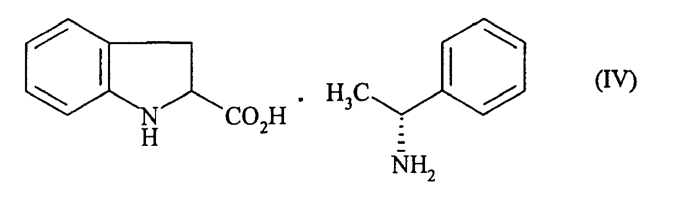
which is filtered off, and there is thus isolated :
• on the one hand the (2S) isomer of formula (IV a) : 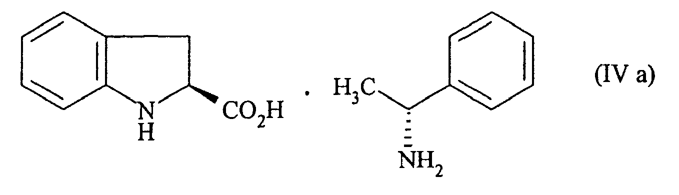 in the form of crystals,
which compound of formula (IV a) is then treated with hydrochloric acid to yield the compound of formula (I),
• and on the other hand, by evaporation of the filtrate, a mixture of the (2S) isomer of formula (IV a) and the (2R) isomer of formula (IV b) in which the (2R) isomer predominates : 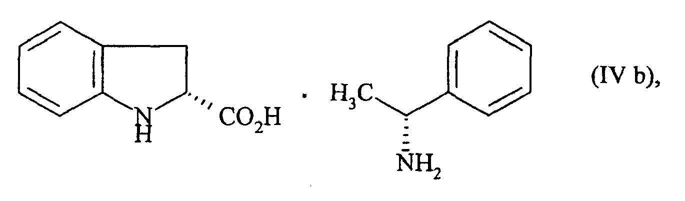 which mixture is then treated with hydrochloric acid to yield a mixture of (2R)-indoline-2-carboxylic acid and (2S)-indoline-2-carboxylic acid in which the (2R) acid predominates,
with which there is repeated, if desired, the series of operations described above,
then, after having carried out from 1 to 6 cycles, all the portions made up of the compound of formula (I) are combined.

3. Synthesis process according to either claim 1 or claim 2, **characterised in that** the chiral amine is (R)-α-methylbenzylamine.

4. Process for the synthesis of perindopril or pharmaceutically acceptable salts thereof starting from the compound of formula (I), **characterised in that** the said compound of formula (I) is obtained according to the process of any one of claims 1 to 3.
